Europäisches Patentamt

European Patent Office

Office européen des brevets

(11) Numéro de publication : **0 440 540 B1**

(12) **FASCICULE DE BREVET EUROPEEN**

(45) Date de publication du fascicule du brevet :
30.03.94 Bulletin 94/13

(51) Int. Cl.⁵ : **C07C 5/27**

(21) Numéro de dépôt : **91400186.2**

(22) Date de dépôt : **28.01.91**

(54) **Procédé d'isomérisation de paraffines normales en présence d'au moins un catalyseur à base d'une zéolithe oméga particulière.**

(30) Priorité : **02.02.90 FR 9001320**

(43) Date de publication de la demande :
**07.08.91 Bulletin 91/32**

(45) Mention de la délivrance du brevet :
**30.03.94 Bulletin 94/13**

(84) Etats contractants désignés :
**BE CH DE ES FR GB IT LI LU NL SE**

(56) Documents cités :
**US-A- 4 727 217**

(73) Titulaire : **INSTITUT FRANCAIS DU PETROLE**
**4, Avenue de Bois Préau**
**F-92502 Rueil-Malmaison (FR)**
Titulaire : **ELF FRANCE, Société Anonyme dite:**
**Tour Elf 2 place de la Coupole La Défense 6**
**F-92400 Courbevoie (FR)**

(72) Inventeur : **Raatz, Francis**
**25, rue de la Carrière**
**F-57500 Saint Avold (FR)**
Inventeur : **Travers, Christine**
**25 bis, rue des Coudreaux**
**F-92500 Rueil Malmaison (FR)**
Inventeur : **Marcilly, Christian**
**91 ter, rue Condorcet**
**F-78800 Houilles (FR)**
Inventeur : **Descourieres, Thierry**
**152, Bd Yves Farge**
**F-69007 Lyon (FR)**
Inventeur : **Fajula, François**
**5, Impasse de Garances**
**F-34820 Teyran (FR)**
Inventeur : **Figueras, François**
**612, rue Jussieu**
**F-44090 Montpellier (FR)**

(74) Mandataire : **Andreeff, François**
**INSTITUT FRANCAIS DU PETROLE 4, avenue de Bois-Préau**
**F-92502 Rueil-Malmaison (FR)**

## Description

La présente invention concerne un procédé d'isomérisation (ou hydroisomérisation) de paraffines normales, en particulier de paraffines normales ayant 4, 5, 6 ou 7 atomes de carbone par molécule, en présence d'au moins un catalyseur à base d'une zéolithe oméga (ou mazzite) particulière.

L'isomérisation des paraffines normales, notamment de faible poids moléculaire, est d'une importance considérable dans l'industrie pétrolière, vu l'indice d'octane particulièrement élevé des isoparaffines.

Il est ainsi intéressant de pouvoir transformer les n-paraffines $C_4$-$C_7$ et surtout $C_5$-$C_6$ en isoparaffines afin d'obtenir un carburant à haut indice d'octane. Ce procédé permet d'améliorer les fractions d'essence légère et, en particulier, les fractions de tête de distillation directe.

Il existe principalement trois types différents de procédés d'isomérisation des paraffines normales :
- les procédés "basse température" (environ 20 à 130°C), utilisant un catalyseur de type Friedel et Crafts, tel que le chlorure d'aluminium,
- les procédés "moyenne température" (environ 150°C), utilisant comme catalyseur un métal supporté tel que le platine sur alumine halogénée,
- les procédés "haute température" (au moins 250°C), mettant en oeuvre des supports zéolithiques généralement associés à un métal hydrogénant du groupe VIII de la classification périodique des éléments.

L'équilibre thermodynamique plus favorable aux isoparaffines à basse température est un avantage des deux premiers types de procédés. Ceux-ci présentent cependant des inconvénients majeurs : les catalyseurs de type Friedel et Crafts et les catalyseurs platine sur alumine halogénée, de par leur nature corrosive, nécessitent des réacteurs en alliages spéciaux très coûteux et sont de plus très sensibles à l'eau et aux traces de soufre.

Les procédés "haute température" font depuis une vingtaine d'années l'objet de nombreux brevets. La plupart des catalyseurs qui y sont décrits sont constitués de zéolithe, en général de mordénite, sous forme acide, avec ou sans promoteur d'hydrogénation.

Il a été ainsi revendiqué des catalyseurs à base de mordénite obtenues à l'aide de traitements spécifiques tels que :
- échange des ions $Na^+$ par les ions $NH_4^+$ ou $H^+$ (US-A-3.190.939),
- le traitement acide :
  . succession acide chaud-$NH_4^+$ (US-A-3.442.794)
  . succession acide chaud-acide froid (US-A-3.475.345)
  . solution d'acide contenant des ions $Na^+$ ou $K^+$ (US-A-2.272.737, 4.359.409, 4.400.576),
- les traitements thermiques à humidité contrôlée (US-A-2.181.928, 3.836.597, 3.842.114).

De même, certains brevets sont relatifs à des procédés de désalumination de mordénite :
- traitements acides sévères : HCl 12N, 100°C (US-A-3.480.539),
- "self-steaming" (calcination en atmosphère confinée) entre 430 et 820°C suivi d'une attaque acide (US-A-3.506.400, 3.551.353).

L'utilisation pour l'isomérisation des paraffines normales d'une mordénite de morphologie particulière, modifiée par une succession de traitements thermiques et de traitements acides, est également décrite dans le brevet US-A-4.727.217.

On a découvert dans la présente invention qu'il est particulièrement avantageux d'utiliser, pour l'isomérisation des paraffines normales et, notamment, des paraffines normales $C_5$-$C_6$, au moins un catalyseur à base d'une zéolithe oméga (ou mazzite) convenablement modifiée, catalyseur qui présente des performances accrues par rapport aux catalyseurs à base de mordénite de l'art antérieur.

La zéolithe oméga (ou mazzite) employée dans la présente invention possède un rapport molaire $SiO_2/Al_2O_3$ compris entre 6,5 et 80, de préférence entre 10 et 40, une teneur pondérale en sodium inférieure à 0,2 %, de préférence inférieure à 0,1 %, par rapport au poids de zéolithe sèche. Elle possède habituellement des paramètres cristallins "a" et "c" respectivement inférieurs ou égaux à 1,814 nm et 0,760 nm (1 nm = $10^{-9}$ m), de préférence respectivement compris entre 1,814 et 1,794 nm et entre 0,760 et 0,749 nm, une capacité d'adsorption d'azote, mesurée à 77K sous une pression partielle égale à 0,19, supérieure à environ 8 % en poids, de préférence supérieure à environ 11 % en poids. Sa répartition poreuse comprend généralement entre 5 et 50 % du volume poreux contenu dans des pores de rayons (mesurés par la méthode BJH) situés entre 1,5 et 14 nm, de préférence entre 2,0 et 8,0 nm (mésopores). D'une façon générale, son taux de cristallinité DX (mesurée d'après son Diffractogramme de rayons X) est supérieur à 60 %.

La zéolithe oméga utilisée dans la présente invention est avantageusement préparée à partir d'une zéolithe oméga obtenue par synthèse (dite zéolithe oméga de départ), possédant un rapport molaire $SiO_2/Al_2O_3$ compris entre 6 et 10, une teneur pondérale en sodium généralement comprise entre 4 et 6,5 % par rapport au poids de zéolithe sèche, un volume poreux déterminé par adsorption d'azote (à 77K sous une pression partielle de 0,19) compris entre 0,05 et 0,15 cm³.g⁻¹ ; la structure cristalline de la zéolithe oméga de départ est constituée d'enchaînements de tétraèdres $SiO_4$ et $AlO_4$ générateurs de gros canaux à ouverture dodécagonale parallèles à l'axe $\vec{c}$. La zéolithe oméga de départ peut ou non contenir une quantité notable de cations organiques suivant qu'elle a été synthétisée en présence ou en absence de cations organiques.

Afin d'obtenir la zéolithe oméga (désaluminée, stabilisée et appauvrie en ions sodium) employée dans la présente invention, la zéolithe oméga de départ est de préférence soumise aux traitements connus suivants :
- Elimination des cations organiques, en particulier des ions tétraméthylammonium, éventuellement présents lors de sa synthèse, par une calcination, sous mélange gaz inerte (azote par exemple) plus air dans un rapport molaire gaz inerte/air compris habituellement entre 3:1 et 7:1, puis généralement sous air pur, à une température habituellement comprise entre 450 et 650°C, de préférence entre 520 et 600°C, pendant une durée comprise en général entre 0,5 et 6 heures, de préférence entre 1 et 4,5 heures
- Elimination des cations sodium par au moins un échange cationique où les cations sodium sont remplacés par des cations ammonium, à une température généralement comprise entre 10 et 150°C, dans une solution d'au moins un sel d'ammonium ionisable (en général le nitrate d'ammonium), de molarité habituellement située entre 0,5 et 20N, de préférence entre 4 et 12N. Le produit obtenu peut être ensuite éventuellement soumis à au moins un lavage à l'eau déminéralisée à température ambiante (10 à 30°C).
- Calcination en présence de vapeur d'eau, l'atmosphère de calcination contenant au moins 1 % et, de préférence, au moins 5 % de vapeur d'eau, à une température généralement comprise entre 300 et 800°C, de préférence entre 400 et 700°C, la durée de ce traitement thermique étant habituellement comprise entre 0,2 et 8 heures, de préférence entre 1 et 6 heures.
- Attaque(s) acide(s) dans une solution d'au moins un acide minéral ou organique fort, généralement HCl ou $HNO_3$, de normalité comprise entre 0,05 et 6N, de préférence entre 0,1 et 4N, à une température habituellement comprise entre 20 et 150°C, de préférence entre 80 et 150°C, pendant une durée en général supérieure à 0,2 heure, de préférence à 1 heure.

Il est possible d'inverser l'ordre élimination des cations organiques-élimination des cations sodium ou encore d'omettre l'étape de décomposition thermique des cations organiques dans le cas où ces derniers ne sont pas présents en quantité notable.

Il est également possible d'effectuer une ou plusieurs séquences calcination en présence de vapeur d'eau-attaque(s) acide(s).

Le catalyseur, à base d'une zéolithe oméga ainsi modifiée, utilisé dans la présente invention renferme habituellement aussi une matrice généralement amorphe choisie par exemple dans le groupe formé par les argiles, les alumines, la silice, la magnésie, la zircone, l'oxyde de titane, l'oxyde de bore et toute combinaison d'au moins deux des composés précités, comme la silice-alumine, la silice-magnésie, l'alumine-oxyde de bore. Le mélange zéolithe oméga + matrice est mis en forme par toute méthode connue de l'homme du métier comme, par exemple, l'extrusion, le pastillage, la dragéification, la coagulation en goutte...La teneur pondérale en zéolithe oméga du support obtenu (zéolithe oméga + matrice) est habituellement compris entre 30 et 98 %,de préférence entre 60 et 90 %.

D'une manière générale, ledit catalyseur renferme en plus de la zéolithe oméga (et éventuellement d'une matrice) au moins un métal du groupe VIII de la classification périodique des éléments, de préférence choisi parmi le groupe formé par le platine, le palladium et le nickel. Le platine et le palladium sont les métaux les plus préférés.

Le métal hydrogénant du groupe VIII, d'une manière préférée le platine et/ou le palladium , est déposé sur le support par tout procédé connu de l'homme du métier. On peut utiliser notamment la technique d'échange cationique avec compétition, où l'agent compétiteur est de préférence le nitrate d'ammonium. Dans le cas du platine ou du palladium, on utilise habituellement un complexe tétrammine du platine ou un complexe tétrammine du palladium ; ces métaux se déposeront alors pratiquement en totalité sur la zéolithe oméga. Cette technique d'échange cationique peut également être utilisée pour déposer directement le métal sur la poudre de zéolithe, avant son mélange éventuel avec une matrice.

On peut également déposer le platine ou la palladium non plus directement sur la zéolithe oméga mais sur l'alumine (si l'alumine est la matrice utilisée), avant ou après l'étape de mise en forme, en mettant en oeuvre un échange anionique avec de l'acide hexachloroplatinique, de l'acide hexachloropalladique et/ou du chlorure de palladium en présence d'un agent compétiteur, par exemple l'acide chlorhydrique.

Le dépôt du métal (ou des métaux) est suivi en général d'une calcination sous air ou oxygène, usuellement entre 300 et 600°C durant 0,5 à 10 heures, de préférence entre 350 et 550°C durant 1 à 4 heures. On peut éventuellement procéder ensuite à une réduction sous hydrogène,par exemple entre 350 et 550°C durant 1 à 4 heures.

La teneur pondérale du catalyseur en métal du groupe VIII est habituellement comprise, dans le cas du platine et du palladium, entre 0,05 et 1 %, de préférence entre 0,1 et 0,6 %, et, dans le cas du nickel, entre 0,1 et 10 %, de préférence entre 0,2 et 5 %.

Selon le procédé de l'invention, la charge contenant des paraffines normales, de préférence des paraffines normales légères à 5 ou 6 atomes de carbone, et de l'hydrogène sont mis en contact d'au moins un catalyseur décrit précédemment dans les conditions d'isomérisation mentionnées ci-dessous. Ce contact peut notamment s'effectuer en utilisant ledit catalyseur en lit(s) fixe(s), en lits(s) fluidisé(s) ou en batch (c'est-à-dire en discontinu).

Le procédé selon l'invention est habituellement mis en oeuvre entre 200 et 300°C et, de préférence, entre 210 et 280°C, à des pressions comprises entre 0,1 et 7 MPa et, de préférence, entre 0,5 et 5 MPa. La vitesse spatiale peut être comprise entre 0,1 et 10, de préférence entre 0,15 et 5, litres d'hydrocarbures liquides par litre de catalyseur et par heure. Le rapport molaire $H_2$/charge est en général compris entre 0,5 et 30 et, de préférence, entre 1 et 25. L'isomérisation étant une réaction équilibrée, l'isomérisat contient généralement encore une quantité assez importante de paraffines normales non converties. Ces paraffines peuvent être séparées des isomères par exemple par distillation ou par fractionnement sur tamis moléculaire et recyclées dans l'unité d'isomérisation utilisée pour effectuer le procédé selon l'invention.

Les exemples suivants illustrent l'invention sans toutefois en limiter la portée.

EXEMPLE 1 : Préparation d'un catalyseur A conforme à celui employé dans l'invention.

La matière première utilisée est une zéolithe oméga synthétisée dans le système (Na, TMA) selon la méthode décrite dans la demande de brevet F R-A-2582234. Cette zéolithe oméga présente, après synthèse, un rapport molaire $SiO_2/Al_2O_3$ de 6,4, une teneur pondérale en sodium de 5,7 % par rapport au poids de zéolithe sèche, un volume poreux déterminé par adsorption d'azote (à 77K sous une pression partielle de 0,19) de 0,062 $cm^3.g^{-1}$, des paramètres cristallins "a" et "c" respectivement égaux à 1,822 et 0,764 nm (1 nm = $10^{-9}$ m).

Cette zéolithe oméga de départ est soumise à une calcination à environ 550°C, d'abord sous un mélange azote+air à raison de 2 $1.h^{-1}$. (g de zéolithe)$^{-1}$ d'azote et 0,4 $1.h^{-1}$.(g de zéolithe)$^{-1}$ d'air pendant 2 heures, puis dans les mêmes conditions sous air pur. Le produit ainsi obtenu subit alors 4 échanges cationiques successifs dans une solution de nitrate d'ammonium 4N, à une température de 100°C, pendant 4 heures environ, le volume (V) de solution engagé étant égal à 4 fois le poids (P) de zéolithe sèche (V/P = 4 $cm^3$/g). Il est ensuite soumis à une calcination en présence de vapeur d'eau, l'atmosphère de calcination contenant 50 % de vapeur d'eau, à une température de 650°C, pendant 4 heures. Puis il subit une attaque acide dans une solution d'acide chlorhydrique 0,8N dont le volume (V') est égal à 10 fois le poids (P') de zéolithe sèche (V'/P' = 10 $cm^3$/g), à une température de 100°C, pendant environ 4 heures.

La zéolithe oméga obtenue possède un rapport molaire $SiO_2/Al_2O_3$ de 30,4, une teneur pondérale en sodium inférieure à 0,06 %, des paramètres cristallins "a" et "c" respectivement égaux à 1,807 nm et 0,755 nm, une capacité d'adsorption d'azote mesurée à 77K sous une pression partielle de 0,19 égale à 17,7 % en poids et un taux de cristallinité DX de 85 %.

Cette zéolithe oméga est ensuite malaxée avec de l'alumine ; le mélange obtenu, contenant 20 % en poids d'alumine, est forcé au travers d'une filière, puis séché et calciné.

Du platine est ensuite déposé sur le support constitué par la zéolithe oméga et l'alumine par échange cationique à partir de chlorure de platine tétrammine $Pt(NH_3)_4Cl_2$, le nitrate d'ammonium étant l'ion compétiteur.

La teneur pondérale en platine du catalyseur A ainsi obtenu est de 0,3 %.

EXEMPLE 2 : Préparation d'un catalyseur B conforme à celui employé dans l'invention.

Le catalyseur B diffère du catalyseur A préparé dans l'exemple 1 en ce que l'attaque acide est réalisée avec une solution d'acide chlorhydrique 0,15N.

La zéolithe oméga obtenue possède un rapport molaire $SiO_2/Al_2O_3$ de 7,2, une teneur pondérale en sodium de 660 ppm, des paramètres cristallins "a" et "c" respectivement égaux à 1,811 nm et 0,753 nm, une capacité d'adsorption d'azote mesurée à 77K sous une pression partielle de 0,19 égale à 12 % en poids et un taux de cristallinité DX de 89 %.

Les étapes de mélange zéolithe-alumine, de mise en forme, de dépôt du platine sont identiques à celles décrites dans l'exemple 1.

La teneur pondérale en platine du catalyseur B ainsi obtenu est de 0,3 %.

EXEMPLE 3 : Préparation d'un catalyseur C conforme à celui employé dans l'invention.

Le catalyseur C diffère du catalyseur A préparé dans l'exemple 1 en ce que l'attaque acide est réalisée avec une solution d'acide chlorhydrique 3N.

La zéolithe oméga obtenue possède un rapport molaire $SiO_2/Al_2O_3$ de 44, une teneur pondérale en sodium de 150 ppm, des paramètres cristallins "a" et "c" respectivement égaux à 1,800 nm et 0,753 nm, une capacité d'adsorption d'azote mesurée à 77K sous une pression partielle de 0,19 égale à 17,7 % en poids et un taux de cristallinité de 63 %.

Les étapes de mélange zéolithe-alumine, de mise en forme, de dépôt du platine sont identiques à celles décrites dans l'exemple 1.

La teneur pondérale en platine du catalyseur C ainsi obtenue est de 0,3 %.

EXEMPLE 4 : Préparation d'un catalyseur D non conforme à celui employé dans l'invention.

La matière première utilisée est une mordénite "petits pores", référence Alite 150 de la Société Chimique de la Grande Paroisse. Sa formule chimique à l'état anhydre est : $Na\, AlO_2(SiO_2)_{5,5}$.

50 grammes de cette poudre sont plongés dans une solution 2M de nitrate d'ammonium et la suspension est portée à 95°C pendant deux heures. Le volume de la solution de nitrate d'ammonium engagée est égal à 4 fois le poids de zéolithe sec (V/P = 4 $cm^3$/g). Cette opération d'échange cationique est recommencée 3 fois. Après le 3ème échange, le produit est lavé à l'eau, à 20°C pendant 20 minutes, avec un rapport V/P égal à 4 $cm^3$/g . La teneur en sodium, exprimée en pourcentage en poids par rapport au poids de solide sec, passe de 5,5 à 0,1 %. Le produit est ensuite filtré et soumis à une calcination en atmosphère confinée ("self-steaming") à 600°C pendant 2 heures (l'atmosphère de calcination contenant au moins 5 % de vapeur d'eau). On procède ensuite à une attaque acide : le solide est porté à reflux dans une solution d'acide chlorhydrique 0,6N à 90°C pendant 2 heures, avec un rapport V/P égal à 8 $cm^3$/g , où V est le volume de la solution d'acide chlorhydrique et P le poids de mordénite sèche. Le produit est ensuite filtré, lavé à l'acide chlorhydrique 0,1N puis à l'eau.

La mordénite ainsi obtenue possède un rapport molaire $SiO_2/Al_2O_3$ de 24. Sa teneur pondérale en sodium est d'environ 300 ppm. Son volume de maille élémentaire est égal à 2,75 $nm^3$.

Les étapes de mélange mordénite-alumine, de mise en forme, de dépôt du platine sont identiques à celles décrites dans l'exemple 1.

La teneur pondérale en platine du catalyseur D ainsi obtenu est de 0,3 %.

EXEMPLE 5 : Test d'isomérisation.

Les catalyseurs préparés dans les exemples précédents sont chacun testés en isomérisation d'une charge de normal hexane. Pour cela, ils sont placés dans une unité catalytique en lit fixe et réduits sous hydrogène à 450°C.

Les conditions opératoires du test d'isomérisation sont les suivantes :

. température : voir tableau I

. pression : 2 MPa

. vitesse spatiale en poids de charge par unité de poids de catalyseur et par heure : 0,2

. rapport molaire $H_2$/charge : 20.

Les performances des catalyseurs A, B, C et D, reportées dans le tableau I, sont définies par :

$$\text{Conversion du n - hexane (\%)} = \frac{\text{Masse de n} - \text{hexane dans la charge} - \text{Masse de n} - \text{hexane dans la recette}}{\text{Masse de n} - \text{hexane dans la charge}} \times 100$$

$$\text{Sélectivité en isomérisation (\%)} = \frac{\Sigma\,(\text{Masse des isomères dans la recette})}{\Sigma\,(\text{Masse des produits de la réaction})} \times 100$$

$$\text{Sélectivité en craquage (\%)} = \frac{\Sigma\,(\text{Masse des produits craqués dans la recette})}{\Sigma\,(\text{Masse des produits de la réaction})} \times 100$$

$$\%\ 22\ DMC4 = \frac{\text{Masse de 22DMC4 dans la recette}}{\Sigma\,(\text{Masse des isomères dans la recette})} \times 100$$

(avec 22DMCA : diméthyl-2,2 butane)

EP 0 440 540 B1

TABLEAU I

|  | A | B | C | D |
|---|---|---|---|---|
| $SiO_2/Al_2O_3$ | 30,4 | 7,2 | 44 | 24 |
| Conversion du n-hexane(%) | 60 | 60 | 60 | 60 |
| Sélectivité en isomérisa-tion (%) | 98,3 | 99,3 | 99,5 | 98,3 |
| Sélectivité en craquage(%) | 1,7 | 0,7 | 0,5 | 1,7 |
| % 22DMC4 | 25 | 10 | 15 | 15 |
| Température (°C) | 215 | 260 | 255 | 230 |

La lecture du tableau I permet d'établir une comparaison, à isoconversion (60 %), entre, d'une part, les résultats obtenus avec les catalyseurs A, B et C, et, d'autre part, les résultats obtenus avec le catalyseur D.

Le catalyseur A présente, par rapport au catalyseur D, une activité sensiblement accrue (température inférieure de 15°C pour atteindre le même taux de conversion du n-hexane) et une sélectivité en 22DMC4, isomère de haut indice d'octane, très supérieure, tout en ayant la même sélectivité en isomérisation.

Les catalyseurs B et C, qui sont un peu moins actifs que le catalyseur D, présentent, même à températures plus élevées, une sélectivité en isomérisation supérieure et une sélectivité en craquage (réaction parasite) très inférieure.

**Revendications**

1. Procédé d'isomérisation de paraffines normales en présence d'au moins un catalyseur à base d'une zéolithe oméga possédant un rapport molaire $SiO_2/Al_2O_3$ compris entre 6,5 et 80, une teneur pondérale en sodium inférieure à 0,2 %, des paramètres cristallins "a" et "c" respectivement inférieurs ou égaux à 1, 814 nm et 0, 760 nm, une capacité d'adsorption d'azote, mesurée à 77K sous une pression partielle de 0,19, supérieure à environ 8 % en poids, ledit catalyseur renfermant en outre au moins un métal du groupe VIII de la classification périodique des éléments.

2. Procédé selon la revendication 1 dans lequel ladite zéolithe oméga possède un rapport molaire $SiO_2/Al_2O_3$ compris entre 10 et 40 et une teneur pondérale en sodium inférieure à 0,1 %.

3. Procédé selon l'une des revendications 1 et 2 dans lequel ladite zéolithe oméga possède des paramètres cristallins " a" et " c " respectivement compris entre 1,814 et 1,794 nm et entre 0,760 et 0,749 nm et une capacité d'adsorption d'azote, mesurée à 77K sous une pression partielle de 0,19, supérieure à environ 11 % en poids.

4. Procédé selon l'une des revendications 1 à 3 dans lequel ladite zéolithe oméga possède une répartition poreuse comprenant entre 5 et 50 % du volume poreux contenu dans des pores de rayons (mesurés par

6

la méthode de BJH) situés entre 1,5 et 14 nm.

5. Procédé selon l'une des revendications 1 à 4 dans lequel ledit catalyseur renferme en outre une matrice.

6. Procédé selon la revendication 5 dans lequel la teneur pondérale en zéolithe oméga du support constitué par la zéolithe oméga et la matrice est comprise entre 30 et 98 %.

7. Procédé selon l'une des revendications 1 à 6 dans lequel ledit métal du groupe VIII est choisi dans le groupe formé par le platine, le palladium et le nickel.

8. Procédé selon la revendication 7 dans lequel la teneur pondérale du catalyseur en métal du groupe VIII est comprise entre 0,05 et 1 % dans le cas où ledit métal est le platine ou le palladium et entre 0,1 et 10 % dans le cas où ledit métal est le nickel.

9. Procédé selon l'une des revendications 7 et 8 dans lequel ledit métal du groupe VIII est choisi dans le groupe formé par le platine et le palladium.

10. Procédé selon l'une des revendications 1 à 9, dans lequel lesdites paraffines normales sont des paraffines normales légères à 5 ou 6 atomes de carbone.


## Patentansprüche

1. Verfahren zur Isomerisierung von Normal-Paraffinen (n-Paraffinen) in Anwesenheit wenigstens eines Katalysators auf der Basis eines Omega-Zeoliths, der ein Molverhältnis $SiO_2/Al_2O_3$ zwischen 6,5 und 80, einen Gehalt an Natrium von weniger als 0,2 Gew.-%, Kristallparameter "a" und "c", die jeweils kleiner oder gleich 1,814 nm und 0,760 nm sind, und ein Stickstoffadsorptionsvermögen, gemessen bei 77K unter einem Partialdruck von 0,19, von größer als etwa 8 Gew.-% hat, wobei dieser Katalysator im übrigen wenigstens ein Metall der Gruppe VIII des Periodensystems der Elemente enthält.

2. Verfahren nach Anspruch 1, bei dem dieser Omega-Zeolith ein Molverhältnis $SiO_2/Al_2O_3$ zwischen 10 und 40 und einen Gehalt an Natrium von weniger als 0,1 Gew.-% hat.

3. Verfahren nach einem der Ansprüche 1 und 2, bei dem dieser Omega-Zeolith Kristallparameter "a" und "c", die jeweils zwischen 1,814 und 1,794 nm und zwischen 0,760 und 0,749 nm liegen sowie ein Stickstoffadsorptionsvermögen, gemessen bei 77K unter einem Partialdruck von 0,19, von mehr als etwa 11 Gew.-% hat.

4. Verfahren nach einem der Ansprüche 1 bis 3, bei dem dieser Omega-Zeolith eine Porenverteilung besitzt, bei der zwischen 5 und 50 % des Porenvolumens in Poren mit Radien (gemessen nach dem BJH-Verfahren) zwischen 1,5 und 14 nm enthalten sind.

5. Verfahren nach einem der Ansprüche 1 bis 4, bei dem dieser Katalysator außerdem eine Matrix enthält.

6. Verfahren nach Anspruch 5, bei dem der Gehalt an Omega-Zeolith des Trägers, der besteht aus dem Omega-Zeolith und der Matrix, zwischen 30 und 98 Gew.-% liegt.

7. Verfahren nach einem der Ansprüche 1 bis 6, bei dem dieses Metall der Gruppe VIII gewählt ist aus der Gruppe, die gebildet wird durch Platin, Palladium und Nickel.

8. Verfahren nach Anspruch 7, bei dem der Gehalt des Katalysators an Metall der Gruppe VIII zwischen 0,05 und 1 Gew.-% liegt für den Fall, daß dieses Metall Platin oder Palladium ist, und zwischen 0,1 und 10 Gew.-% liegt für den Fall, daß dieses Metall Nickel ist.

9. Verfahren nach einem der Ansprüche 7 und 8, bei dem dieses Metall der Gruppe VIII gewählt ist aus der Gruppe, die durch Platin und Palladium gebildet ist.

10. Verfahren nach einem der Ansprüche 1 bis 9, bei dem diese Normal-Paraffine leichte Normal-Paraffine mit 5 oder 6 Kohlenstoffatomen sind.

7

## Claims

1. A process for isomerizing normal paraffins in the presence of at least one catalyst based on an omega zeolite having a molar ratio $SiO_2/Al_2O_3$ ranging from 6.5 to 80, a content by weight of sodium lower than 0.2 %, crystalline parameters "a" and "c" respectively smaller than or equal to 1.814 nm and 0.760 nm, a capacity of adsorption of nitrogen, measured at 77K under a partial pressure of 0.19, higher than about 8 % by weight, said catalyst also comprising at least one metal from group VIII of the periodic table of elements.

2. A process according to claim 1 wherein said omega zeolite has a molar ratio $SiO_2/Al_2O_3$ ranging from 10 to 40 and a content by weight of sodium lower than 0.1 %.

3. A process according to any one of claims 1 and 2 wherein said omega zeolite has crystalline parameters "a" and "c" respectively ranging from 1.814 to 1.794 nm and from 0.760 to 0.749 nm and a capacity of adsorption of nitrogen, measured at 77K under a partial pressure of 0.19, higher than about 11 % by weight.

4. A process according to any one of claims 1 to 3 wherein said omega zeolite has a pore distribution comprising 5 to 50% of the pore volume contained in pores (measured by the BJH method) with a radius ranging from 1.5 to 14 nm.

5. A process according to any one of claims 1 to 4 wherein said catalyst also comprises a matrix.

6. A process according to claim 5 wherein the content by weight of omega zeolite in the support consisting of the omega zeolite and the matrix ranges from 30 to 98 %.

7. A process according to any one of claims 1 to 6 wherein said metal from group VIII is selected from the group consisting of platinum, palladium and nickel.

8. A process according to claim 7 wherein the content by weight of metal from group VIII in the catalyst ranges from 0.05 to 1 % in the case where said metal is platinum or palladium and from 0.1 to 10 % in the case where said metal is nickel.

9. A process according to any one of claims 7 and 8 wherein said metal from group VIII is selected from the group consisting of platinum and palladium.

10. A process according to any one of claims 1 to 9, wherein said normal paraffins are normal paraffins containing 5 or 6 carbon atoms.